# FASCICULE DE BREVET EUROPEEN

(11) **EP 0 658 094 B1**
(45) Date de publication et mention de la délivrance du brevet: **06.05.1999**
(21) Numéro de dépôt: 94918924.5
(22) Date de dépôt: 14.06.1994
(51) Int. Cl.: A61K 7/13

(54) **COMPOSITION DE TEINTURE D'OXYDATION DES FIBRES KERATINIQUES COMPRENANT UN PARA-AMINOPHENOL, UN META-AMINOPHENOL ET UN ORTHO-AMINOPHENOL, ET PROCEDE DE TEINTURE UTILISANT UNE TELLE COMPOSITION**
ZUSAMMENSETZUNG ZUM OXIDATIVEN FÄRBEN VON KERATINFASERN, DIE EIN P-AMINOPHENOL, EIN M-AMINOPHENOL UND EIN O-AMINOPHENOL ENTHÄLT, UND EIN VERFAHREN ZUM FÄRBEN UNTER VERWENDUNG DIESER ZUSAMMENSETZUNG
OXIDATIVE KERATIN FIBRE DYEING COMPOSITION CONTAINING A PARA-AMINOPHENOL, A META-AMINOPHENOL AND AN ORTHO-AMINOPHENOL, AND DYEING METHOD USING SAID COMPOSITION

(30) Priorité: 16.06.1993 FR 9307238
(43) Date de publication de la demande: 21.06.1995
(73) Titulaire: L'OREAL, 75008 Paris (FR)
(72) Inventeur: COTTERET, Jean, F-78480 Verneuil-sur-Seine (FR); AUDOUSSET, Marie, Pascale, F-92300 Levallois-Perret (FR); LAGRANGE, Alain, F-77700 Coupvray (FR); VANDENBOSCHE, Jean-Jacques, F-93270 Sevran (FR)
(74) Mandataire: Casalonga, Axel
(86) Numéro de dépôt international: FR9400709
(87) Numéro de publication internationale: WO9428864

(56) Documents cités:
- EP-A- 0 366 542
- EP-A- 0 459 901
- WO-A-93/00066
- GB-A- 2 018 808
- GB-A- 2 054 663

## Description

La présente invention est relative à une composition de teinture des fibres kératiniques, et en particulier des fibres kératiniques humaines, comprenant, en association, au moins un para-aminophénol, au moins un 5-aminophénol 2-substitué et au moins un orthoaminophénol, et au procédé de teinture utilisant une telle composition et mettant en oeuvre la révélation par un agent oxydant.

Il est connu de teindre les fibres kératiniques et en particulier les cheveux humains, avec des compositions tinctoriales contenant des précurseurs de colorants d'oxydation, en particulier des ortho- ou paraphénylènediamines, des ortho- ou para-aminophénols, généralement appelés "bases d'oxydation", et des coupleurs encore appelés modificateurs de coloration, plus particulièrement des méta-phénylènediamines aromatiques, des méta-aminophénols et des méta-diphénols, qui permettent de modifier et d'enrichir de reflets les colorations "de fond" obtenues par les produits de condensation des bases d'oxydation.

On recherche, dans le domaine de la teinture capillaire d'oxydation, des précurseurs de colorants d'oxydation ainsi que des coupleurs qui permettent de conférer aux cheveux une coloration ayant une résistance satisfaisante à la lumière, aux lavages, aux intempéries, à la transpiration et aux différents traitements que peuvent subir les cheveux, et d'obtenir un large éventail de nuances de coloration.

Le 3-méthyl para-aminophénol ainsi que son utilisation dans des compositions tinctoriales pour fibres kératiniques en association avec le 2-méthyl 5-aminophénol à titre de coupleur et la para-phénylènediamine ou le 2,5-diaminotoluène, sont connus et décrits dans le brevet US-4.883.656.

Le para-aminophénol et son utilisation dans des compositions tinctoriales pour fibres kératiniques en association avec l'ortho-aminophénol et le 2-méthyl-5-N-β-hydroxyéthylaminophénol à titre de coupleur sont décrits dans les demandes de brevet GB-A-2 018 808 et GB-A-2 054 663.

Cependant, de telles associations ne procurent pas, après application sur les fibres kératiniques, une coloration suffisamment résistante.

La demanderesse vient de découvrir, ce qui fait l'objet de l'invention, que l'utilisation du 3-méthyl para-aminophénol, du 2-méthyl para-aminophénol et/ou du 2-hydroxyméthyl para-aminophénol à titre de précurseurs de colorants d'oxydation, en association avec, à titre de coupleur, un 5-aminophénol substitué en position 2 choisi parmi les 2-méthyl 5-aminophénols de formule (I) définie ci-après et, à titre de précurseur de colorant d'oxydation, un ortho-aminophénol choisi parmi l'ortho-aminophénol et le 3-acétylamino 6-aminophénol, permet d'obtenir, en présence d'un agent oxydant, en milieu acide ou alcalin, après application sur les fibres kératiniques et en particulier les cheveux humains, des colorations aux nuances chaudes et cuivrées et présentant une résistance à la lumière, aux lavages, aux intempéries, à la transpiration et aux différents traitements que peuvent subir les cheveux, particulièrement remarquable et supérieure à celles de l'art antérieur.

La présente invention a donc pour objet une composition de teinture des fibres kératiniques, en particulier des fibres kératiniques humaines telles que les cheveux, comprenant, dans un milieu approprié pour la teinture :
- au moins un précurseur de colorant d'oxydation choisi parmi le 3-méthyl para-aminophénol, le 2-méthyl para-aminophénol et le 2-hydroxyméthyl para-aminophénol, et leurs sels d'addition avec un acide;
- au moins un coupleur choisi parmi les 2-méthyl 5-aminophénols de formule (I) ci-après : 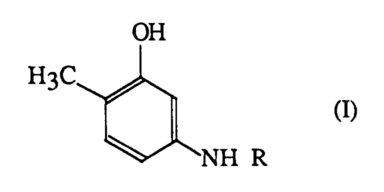 dans laquelle R désigne un atome d'hydrogène, un radical méthyle ou éthyle, ou un groupement β-hydroxyéthyle ou γ-hydroxypropyle, ainsi que leurs sels d'addition avec un acide; et
- au moins, à titre de précurseur de colorant d'oxydation, un ortho-aminophénol choisi parmi l'ortho-aminophénol et le 3-acétylamino 6-aminophénol, ou l'un de leurs sels d'addition avec un acide.

L'invention a également pour objet un agent de teinture à plusieurs composants, dont le premier composant contient les précurseurs de colorants d'oxydation et le coupleur définis ci-dessus et le deuxième composant un agent oxydant.

Un autre objet de l'invention porte sur la composition prête à l'emploi, contenant les différents agents utilisés pour la teinture des fibres kératiniques définis ci-dessus et un agent oxydant, en milieu alcalin ou acide.

L'invention vise également un procédé de teinture des fibres kératiniques, et en particulier des fibres kératiniques humaines telles que les cheveux, consistant à appliquer sur ces fibres :
- au moins un précurseur de colorant d'oxydation choisi parmi le 3-méthyl para-aminophénol, le 2-méthyl para-aminophénol et le 2-hydroxyméthyl para-aminophénol, et leurs sels d'addition avec un acide;
- au moins un coupleur choisi parmi les 2-méthyl 5-aminophénols de formule (I) ci-dessus, ainsi que leurs sels d'addition avec un acide;
- au moins, à titre de précurseur de colorant d'oxydation, un ortho-aminophénol choisi parmi l'ortho-aminophénol et le 3-acétylamino 6-aminophénol, ou l'un de leurs sels d'addition avec un acide;
   la couleur étant révélée à pH acide ou alcalin, à l'aide d'un agent oxydant.

Selon l'invention et parmi les précurseurs de type para ci-dessus, le 3-méthyl p-aminophénol est préféré.

Parmi les coupleurs mentionnés ci-dessus, le 2-méthyl 5-aminophénol est préféré pour être utilisé selon l'invention.

Selon le procédé conforme à l'invention, on applique sur les fibres kératiniques humaines, au moins une composition (A) contenant dans un milieu approprié pour la teinture :
- au moins un précurseur de colorant d'oxydation choisi parmi le 3-méthyl para-aminophénol, le 2-méthyl para-aminophénol, le 2-hydroxyméthyl para-aminophénol, et leurs sels;
- au moins un coupleur choisi parmi les 2-méthyl 5-aminophénols de formule (I) ci-dessus, et leurs sels;
- au moins un ortho-aminophénol choisi parmi l'ortho-aminophénol et le 3-acétylamino 6-aminophénol, ou l'un de leurs sels;
   la couleur étant révélée en milieu acide ou alcalin, à l'aide d'un agent oxydant ajouté juste au moment de l'emploi à la composition (A) ou présent dans une composition (B) appliquée simultanément ou séquentiellement de façon séparée.

L'invention a également pour objet des dispositifs de teinture ou "kits", à plusieurs compartiments, permettant de mettre en oeuvre le procédé indiqué ci-dessus.

Un tel kit de teinture comporte au moins deux compartiments, dont le premier renferme la composition (A) telle que définie ci-dessus et le second renferme la composition (B) comprenant un agent oxydant dans un milieu approprié pour la teinture.

D'autres objets de l'invention apparaîtront à la lecture de la description et des exemples qui suivent.

Les sels d'acide utilisés selon l'invention sont choisis de préférence parmi les chlorhydrates, les sulfates, les bromhydrates et les tartrates.

Le 3-méthyl para-aminophénol, le 2-méthyl para-aminophénol et le 2-hydroxyméthyl para-aminophénol ou leurs sels, sont présents à une concentration totale de 0,01% à 4% en poids par rapport au poids total de la composition tinctoriale, et de préférence de 0,1 à 2% en poids.

Les 2-méthyl 5-aminophénols de formule (I) ci-dessus ainsi que leurs sels, représentent au total de 0,005% à 5% en poids du poids total de la composition tinctoriale, et de préférence de 0,01 à 3,5% en poids.

L'ortho-aminophénol ou le 3-acétylamino 6-aminophénol représentent de 0,1% à 2% en poids par rapport au poids total de la composition tinctoriale, et de préférence de 0,2 à 1% en poids.

L'ensemble précurseurs de colorants d'oxydation et coupleurs selon l'invention, représente de 0,3 à 10% en poids, et de préférence de 0,4 à 5% en poids, par rapport au poids total de la composition.

L'agent oxydant est choisi de préférence parmi le peroxyde d'hydrogène, le peroxyde d'urée, les bromates de métaux alcalins, les persels tels que les perborates et les persulfates. Le peroxyde d'hydrogène est particulièrement préféré.

La composition (A), qui renferme l'association des colorants telle que décrite ci-dessus, peut avoir un pH compris entre 3 et 10,5, qui peut être ajusté à la valeur choisie au moyen d'agents alcalinisants habituellement utilisés en teinture des fibres kératiniques, tels que l'ammoniaque, les carbonates alcalins, les alcanolamines, par exemple les mono-, di- et triéthanolamines ainsi que leurs dérivés, les hydroxydes de sodium ou de potassium, les composés de formule : 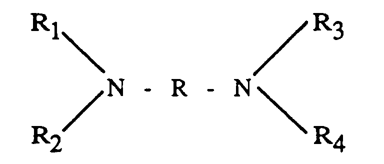 dans laquelle :
R est un reste propylène éventuellement substitué par un groupement hydroxyle ou un radical alkyle en C₁-C₄;
R₁, R₂, R₃ et R₄, simultanément ou indépendamment l'un de l'autre, représentent un atome d'hydrogène, un radical alkyle en C₁-C₄ ou hydroxyalkyle en C₁-C₄;
   ou d'agents acidifiants classiques, tels que les acides minéraux ou organiques, par exemple les acides chlorhydrique, tartrique, citrique et phosphorique.

Le pH de la composition (B) renfermant l'agent oxydant tel que défini ci-dessus, est tel qu'après un mélange avec la composition (A), le pH de la composition appliquée sur les fibres kératiniques humaines varie de préférence entre 3 et 11. Il est ajusté à la valeur désirée à l'aide d'agents acidifiants ou éventuellement alcalinisants bien connus de l'état de la technique, tels que décrits ci-dessus.

La composition oxydante (B) est de préférence constituée par une solution d'eau oxygénée.

Selon un mode de réalisation préféré du procédé de teinture de l'invention, on mélange au moment de l'emploi la composition tinctoriale (A) décrite ci-dessus avec une solution oxydante en une quantité suffisante pour développer une coloration. Le mélange obtenu est ensuite appliqué sur les fibres kératiniques humaines et on laisse poser pendant 5 à 40 minutes, de préférence 15 à 30 minutes, après quoi on les rince, les lave au shampooing, on les rince à nouveau et on les sèche.

Selon l'invention, les compositions tinctoriales peuvent contenir, en plus des colorants définis ci-dessus, d'autres coupleurs et/ou des colorants directs, notamment en vue de nuancer ou d'enrichir en reflets les colorations apportées par les précurseurs de colorants d'oxydation.

Ces coupleurs sont bien connus en eux-mêmes et sont choisis parmi les composés benzéniques portant au moins 2 substitutions en position méta l'une par rapport à l'autre et différents des 2-méthyl 5-aminophénols de formule (I) ci-dessus, ainsi que leurs sels; l'α-naphtol; les dérivés indoliques; les coupleurs possédant un groupement méthylène actif, tels que les composés β-cétoniques; les pyrazolones, ainsi que leurs sels.

Les colorants directs sont de préférence des colorants azoïques, anthraquinoniques ou des dérivés nitrés de la série benzénique.

Les compositions tinctoriales conformes à l'invention contiennent également dans leur forme de réalisation préférée, des agents tensioactifs anioniques, cationiques, non-ioniques, amphotères ou leurs mélanges. Parmi ces agents tensio-actifs, on peut citer les alkylbenzènesulfonates, les alkylnaphtalènesulfonates, les sulfates, les éthersulfates et les sulfonates d'alcools gras, les alkylpolyglycosides, les sels d'ammonium quaternaire, tels que le bromure de triméthylcétylammonium, le bromure de cétylpyridinium, les éthanolamides d'acides gras éventuellement oxyéthylénés, les acides, alcools et amines polyoxyéthylénés, les alcools gras polyglycérolés, les alkylphénols polyoxyéthylénés ou polyglycérolés, ainsi que les alkylsulfates polyoxyéthylénés.

Ces agents tensio-actifs sont présents dans les compositions conformes à l'invention dans des proportions comprises entre 0,5 et 55 % en poids, et de préférence entre 2 et 50 % en poids par rapport au poids total de la composition.

Ces compositions peuvent également contenir des solvants organiques pour solubiliser les composants qui ne seraient pas suffisamment solubles dans l'eau. Parmi ces solvants, on peut citer à titre d'exemple, les alcanols inférieurs en C₁-C₄, tels que l'éthanol et l'isopropanol; le glycérol; les glycols ou éthers de glycols comme le 2-butoxyéthanol, le propylèneglycol, le monoéthyléther et le monométhyléther du diéthylèneglycol, ainsi que les alcools aromatiques comme l'alcool benzylique ou le phénoxyéthanol, les produits analogues et leurs mélanges.

Les solvants sont présents de préférence dans des proportions comprises entre 1 et 40 % en poids, et en particulier entre 5 et 30 % en poids par rapport au poids total de la composition.

Les agents épaississants que l'on peut ajouter dans les compositions conformes à l'invention, peuvent être choisis parmi l'alginate de sodium, la gomme arabique, les polymères d'acide acrylique éventuellement réticulés, les dérivés de cellulose, les hétérobiopolysaccharides tels que la gomme de xanthane, on peut également utiliser des agents épaississants minéraux tels que la bentonite.

Ces agents épaississants sont présents de préférence dans des proportions comprises entre 0,1 et 5 %, et en particulier entre 0,2 et 3 % en poids par rapport au poids total de la composition.

Les agents antioxydants qui peuvent être présents dans les compositions sont choisis en particulier parmi le sulfite de sodium, l'acide thioglycolique, l'acide thiolactique, le bisulfite de sodium, l'acide déhydroascorbique, l'hydroquinone, la 2-méthyl hydroquinone, la tertiobutyl hydroquinone et l'acide homogentisique.

Ces agents antioxydants sont présents dans la composition dans des proportions comprises entre 0,05 et 1,5 % en poids par rapport au poids total de la composition.

Ces compositions peuvent également contenir d'autres adjuvants cosmétiquement acceptables, tels que par exemple des agents de pénétration, des agents séquestrants, des parfums, des tampons, des agents dispersants, des agents de traitement, des agents de conditionnement, des agents filmogènes, des agents conservateurs, des agents opacifiants.

La composition appliquée sur les cheveux peut se présenter sous des formes diverses, telles que sous forme de liquide, de crème, de gel, ou sous toute autre forme appropriée pour réaliser une teinture des fibres kératiniques, et notamment des cheveux humains. Ces compositions peuvent être conditionnées sous pression en flacons aérosols en présence d'un agent propulseur et former des mousses.

Les exemples qui suivent sont destinés à illustrer l'invention sans pour autant présenter un caractère limitatif.

### EXEMPLE 1

On prépare la composition tinctoriale suivante :

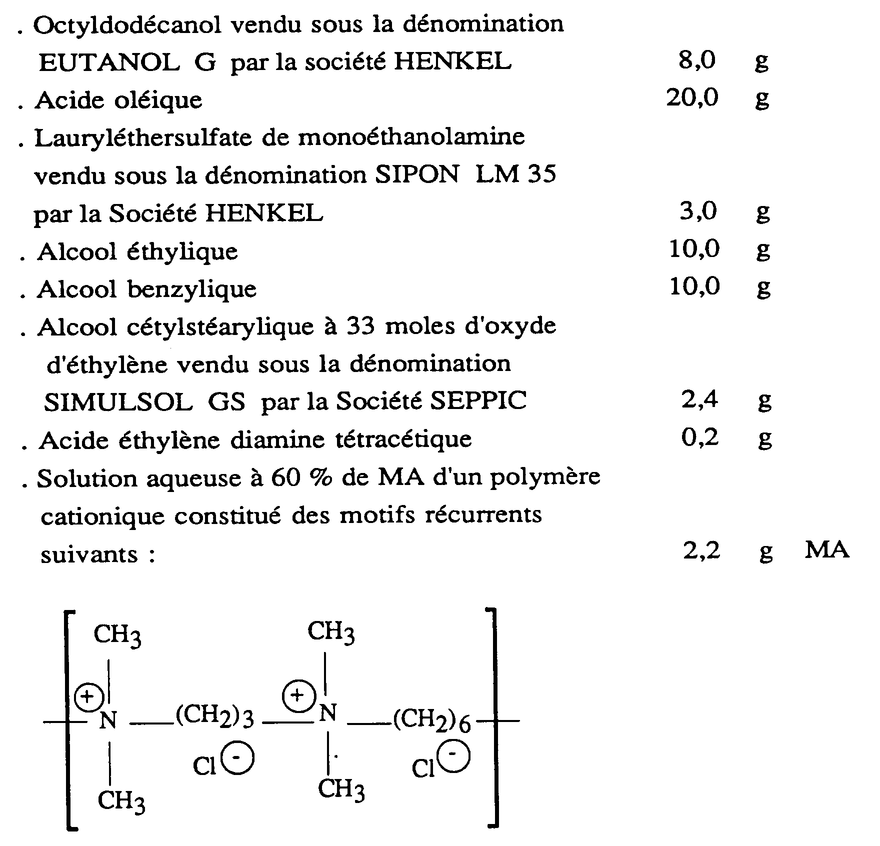

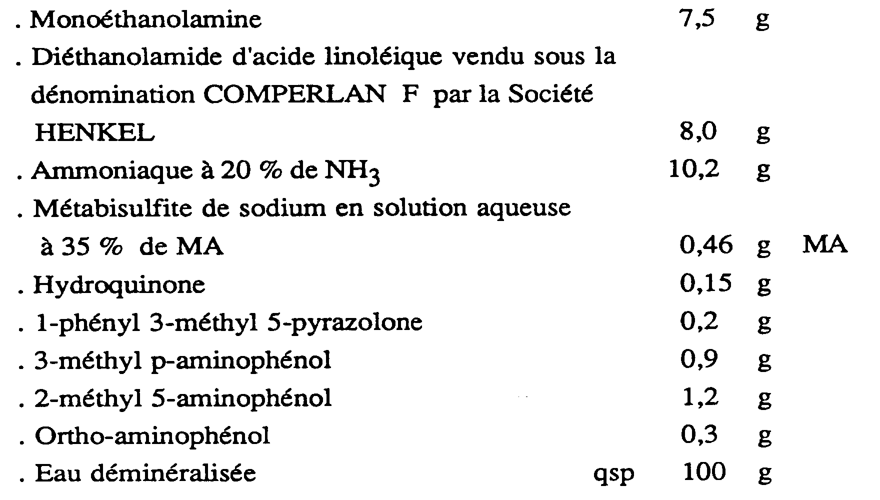

Au moment de l'emploi, on mélange cette composition poids pour poids avec de l'eau oxygénée titrant 20 volumes (6% en poids), de pH 3.

On obtient un mélange de pH 9,8.

On applique ce mélange sur des cheveux gris naturels à 90% de blancs pendant 30 minutes. Après rinçage, lavage au shampooing, rinçage et séchage, les cheveux sont teints en cuivré doré.

### EXEMPLES 2 à 4

On prépare la composition tinctoriale suivante :

| | |
|---|---|
| . Alcool oléique polyglycérolé à 2 moles de glycérol | 4,0 g |
| . Alcool oléique polyglycérolé à 4 moles de glycérol (78% de MA) | 5,69 g MA |
| . Acide oléique | 3,0 g |
| . Amine oléique à 2 moles d'oxyde d'éthylène, vendue sous la dénomination ETHOMEEN O12 par la Société AKZO | 7,0 g |
| . Laurylamino succinamate de diéthylaminopropyle, sel de sodium à 55% de MA | 3,0 g MA |
| . Alcool oléique | 5,0 g |
| . Diéthanolamide d'acide oléique | 12,0 g |
| . Propylèneglycol | 3,5 g |
| . Alcool éthylique | 7,0 g |
| . Dipropylèneglycol | 0,5 g |
| . Monométhyléther de propylèneglycol | 9,0 g |
| . Métabisulfite de sodium en solution aqueuse à 35% de MA | 0,455g MA |
| . Acétate d'ammonium | 0,8 g |
| . Antioxydant, séquestrant qs | |
| . Parfum, conservateur qs | |
| . Ammoniaque à 20% de NH₃ | 10,0 g |
| . Colorants | x g |
| . Eau déminéralisée qsp | 100 g |

Au moment de l'emploi, on mélange cette composition poids pour poids avec de l'eau oxygénée titrant 20 volumes (6% en poids), de pH 3.

On obtient un mélange de pH indiqué dans le tableau ci-après.

On applique ce mélange sur des cheveux gris à 90% de blancs, naturels ou permanentés, pendant 30 minutes. Après rinçage, lavage au shampooing, rinçage et séchage, les cheveux sont teints dans les nuances indiquées dans le tableau ci-après.

**TABLEAU**

| Exemple | 2 | 3 | 4 | 5 |
|---|---|---|---|---|
| 3-méthyl p-aminophénol | 1,5 g | 1,0 g | | 1,0 g |
| 2-méthyl p-aminophénol | | | 0,5 g | |
| 2-hydroxyméthyl p-aminophénol | | | 0,5 g | |
| 2-méthyl 5-aminophénol | 2,0 g | 1,3 g | | |
| 2-méthyl 5-N-(β-hydroxyéthyl) aminophénol | | | 1,3 g | |
| 2-méthyl-5-N-méthylaminophénol | | | | 0,5 g |
| 2-méthyl-5-N-éthylaminophénol | | | | 0,5 g |
| Ortho-aminophénol | 0,7 g | | 0,5 g | 0,5 g |
| 3-acétylamino 6-aminophénol, chlorhydrate | | 0,5 g | | |
| pH du mélange | 9,6 | 9,6 | 9,6 | 9,6 |

| **NUANCE OBTENUE** : | | | | |
|---|---|---|---|---|
| sur cheveux gris naturels à 90% blancs | | Blond clair irisé cuivre | | Cuivré doré |
| sur cheveux gris permanentés à 90% blancs | Blond foncé cuivré rouge intense | | Blond cuivré doré | |

## Revendications

1. Composition tinctoriale pour fibres kératiniques, en particulier pour fibres kératiniques humaines telles que les cheveux, caractérisée par le fait qu'elle comprend, dans un milieu approprié pour la teinture :
- au moins un précurseur de colorant d'oxydation choisi parmi le 3-méthyl para-aminophénol, le 2-méthyl para-aminophénol et le 2-hydroxyméthyl para-aminophénol, et leurs sels d'addition avec un acide;
- au moins un coupleur choisi parmi les 2-méthyl 5-aminophénols de formule (I) ci-après : 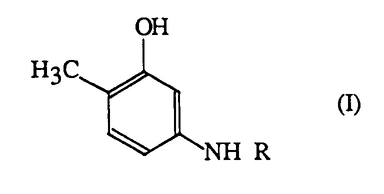 dans laquelle R désigne un atome d'hydrogène, un radical méthyle ou éthyle, ou un groupement β-hydroxyéthyle ou γ-hydroxypropyle, et leurs sels d'addition avec un acide; et
- au moins, à titre de précurseur de colorant d'oxydation, un ortho-aminophénol choisi parmi l'ortho-aminophénol et le 3-acétylamino 6-aminophénol ou l'un de leurs sels d'addition avec un acide.

2. Composition tinctoriale selon la revendication 1, caractérisée par le fait que le précurseur de colorant d'oxydation de type para est le 3-méthyl para-aminophénol ou l'un de ses sels d'addition avec un acide.

3. Composition tinctoriale selon la revendication 1 ou 2, caractérisée par le fait que le coupleur est le 2-méthyl 5-aminophénol ou l'un de ses sels d'addition avec un acide.

4. Composition tinctoriale selon l'une quelconque des revendications 1 à 3, caractérisée par le fait que les sels d'addition avec un acide sont choisis parmi les chlorhydrates, les sulfates, les bromhydrates et les tartrates.

5. Composition tinctoriale selon l'une quelconque des revendications 1 à 4, caractérisée par le fait que le 3-méthyl para-aminophénol, le 2-méthyl para-aminophénol et le 2-hydroxyméthyl para-aminophénol ou leurs sels, sont présents dans une concentration totale de 0,01% à 4% en poids, et de préférence de 0,1 à 2% en poids par rapport au poids total de la composition.

6. Composition tinctoriale selon l'une quelconque des revendications 1 à 5, caractérisée par le fait que les 2-méthyl 5-amino-phénols de formule (I) ou leurs sels, sont présents à une concentration totale de 0,005 à 5% en poids, et de préférence de 0,01 à 3,5% en poids par rapport au poids total de la composition.

7. Composition tinctoriale selon l'une quelconque des revendications 1 à 6, caractérisée par le fait que l'ortho-aminophénol et le 3-acétylamino 6-aminophénol sont présents à une concentration totale de 0,1 à 2% en poids, et de préférence de 0,2 à 1% en poids par rapport au poids total de la composition.

8. Composition tinctoriale selon l'une quelconque des revendications 1 à 7, caractérisée par le fait que les précurseurs de colorants d'oxydation et les coupleurs sont présents dans une concentration totale de 0,3 à 10% en poids, et de préférence de 0,4 à 5% en poids, par rapport au poids total de la composition.

9. Composition tinctoriale selon l'une quelconque des revendications 1 à 8, caractérisée par le fait qu'elle a un pH compris entre 3 et 10,5, ajusté à l'aide d'agents alcalinisants tels que l'ammoniaque, les carbonates alcalins, les alcanolamines, les composés de formule : 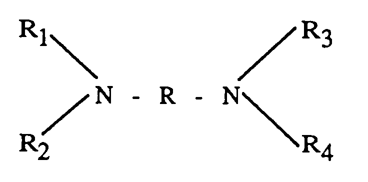 dans laquelle :
R est un reste propylène éventuellement substitué par un groupement hydroxyle ou un radical alkyle en C₁-C₄;
R₁, R₂, R₃ et R₄, simultanément ou indépendamment l'un de l'autre, représentent un atome d'hydrogène, un radical alkyle en C₁-C₄ ou hydroxyalkyle en C₁-C₄; les hydroxydes de sodium et de potassium; ou d'agents acidifiants tels que les acides chlorhydrique, tartrique, citrique et phosphorique.

10. Composition tinctoriale selon l'une quelconque des revendications 1 à 9, caractérisée par le fait qu'elle contient d'autres coupleurs choisis parmi l'α-naphtol, les dérivés indoliques, les composés β-cétoniques et les pyrazolones, ainsi que leurs sels.

11. Composition tinctoriale selon l'une quelconque des revendications 1 à 10, caractérisée par le fait qu'elle contient en outre des colorants directs choisis parmi les colorants azoïques, anthraquinoniques et les dérivés nitrés de la série benzénique.

12. Composition tinctoriale selon l'une quelconque des revendications 1 à 11, caractérisée par le fait qu'elle contient en outre au moins un adjuvant choisi parmi les agents tensio-actifs anioniques, cationiques, non-ioniques, amphotères ou leurs mélanges en des proportions comprises entre 0,5 et 55% en poids, les solvants organiques en des proportions comprises entre 1 et 40% en poids, les agents épaississants en des proportions comprises entre 0,1 et 5% en poids, les agents antioxydants dans des proportions comprises entre 0,05 et 1,5% en poids, les proportions étant calculées par rapport au poids total de la composition, les agents de pénétration, les agents séquestrants, les parfums, les tampons, les agents dispersants, les agents de conditionnement, les agents filmogènes, les agents conservateurs et les agents opacifiants.

13. Composition de teinture des fibres kératiniques, et en particulier des fibres kératiniques humaines telles que les cheveux, selon l'une quelconque des revendications 1 à 12, prête à l'emploi, caractérisée par le fait qu'elle contient en outre un agent oxydant et qu'elle possède un pH compris entre 3 et 11.

14. Composition prête à l'emploi selon la revendication 13, caractérisée par le fait que l'agent oxydant est une solution d'eau oxygénée.

15. Agent de teinture des fibres kératiniques et en particulier des fibres kératiniques humaines telles que les cheveux, caractérisé par le fait qu'il comporte au moins deux composants : un composant (A) constitué par une composition de teinture selon l'une quelconque des revendications 1 à 12, et un composant (B) comprenant un agent oxydant dans un milieu approprié pour la teinture.

16. Agent de teinture selon la revendication 15, caractérisé par le fait que l'agent oxydant est choisi parmi le peroxyde d'hydrogène, le peroxyde d'urée, les bromates de métaux alcalins, les perborates et les persulfates.

17. Procédé de teinture des fibres kératiniques et en particulier des fibres kératiniques humaines telles que les cheveux, caractérisé par le fait qu'il consiste à appliquer sur les fibres une composition tinctoriale (A) selon l'une quelconque des revendications 1 à 12, et à révéler la couleur en milieu acide ou alcalin à l'aide d'un agent oxydant ajouté juste au moment de l'emploi à cette composition ou présent dans une composition (B) appliquée simultanément ou séquentiellement de façon séparée.

18. Procédé de teinture selon la revendication 17, caractérisé par le fait qu'on mélange au moment de l'emploi la composition tinctoriale selon l'une quelconque des revendications 1 à 12 avec une solution oxydante en une quantité suffisante pour développer une coloration, puis on applique le mélange obtenu sur les fibres, on laisse poser pendant 5 à 40 minutes, de préférence 15 à 30 minutes, puis on rince, on lave au shampooing, on rince à nouveau et on sèche.

19. Dispositif à plusieurs compartiments ou kit de teinture, caractérisé par le fait qu'il comporte au moins deux compartiments dont un premier compartiment renferme la composition (A) telle que définie dans l'une quelconque des revendications 1 à 12, et le second compartiment renferme la composition (B) comprenant un agent oxydant dans un milieu approprié pour la teinture.

## Claims

1. Dyeing composition for keratinous fibres, in particular human keratinous fibres such as hair, characterized in that it comprises, in a suitable dyeing medium:
- at least one oxidation dye precursor chosen from 3-methyl-para-aminophenol, 2-methyl-para-aminophenol and 2-hydroxymethyl-para-aminophenol and their addition salts with an acid;
- at least one coupling agent chosen from the 2-methyl-5-aminophenols of formula (I) below: 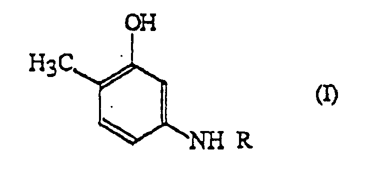 in which R denotes a hydrogen atom, a methyl or ethyl radical or a β-hydroxyethyl or γ-hydroxypropyl group, and their addition salts with an acid; and
- at least one ortho-aminophenol, as oxidation dye precursor, chosen from ortho-aminophenol and 3-acetylamino-6-aminophenol, or one of their addition salts with an acid.

2. Dyeing composition according to Claim 1, characterized in that the oxidation dye precursor of para type is 3-methyl-para-aminophenol or one of its addition salts with an acid.

3. Dyeing composition according to Claim 1 or 2, characterized in that the coupling agent is 2-methyl-5-aminophenol or one of its addition salts with an acid.

4. Dyeing composition according to any one of Claims 1 to 3, characterized in that the addition salts with an acid are chosen from hydrochlorides, sulphates, hydrobromides and tartrates.

5. Dyeing composition according to any one of Claims 1 to 4, characterized in that 3-methyl-para-aminophenol, 2-methyl-para-aminophenol and 2-hydroxymethyl-para-aminophenol or their salts are present in a total concentration of 0.01 % to 4 % by weight, and preferably from 0.1 to 2 % by weight, relative to the total weight of the composition.

6. Dyeing composition according to any one of Claims 1 to 5, characterized in that the 2-methyl-5-aminophenols of formula (I) or their salts are present at a total concentration of 0.005 to 5 % by weight, and preferably from 0.01 to 3.5 % by weight, relative to the total weight of the composition.

7. Dyeing composition according to any one of Claims 1 to 6, characterized in that ortho-aminophenol and 3-acetylamino-6-aminophenol are present at a total concentration of 0.1 to 2 % by weight, and preferably from 0.2 to 1 % by weight, relative to the total weight of the composition.

8. Dyeing composition according to any one of Claims 1 to 7, characterized in that the oxidation dye precursors and the coupling agents are present in a total concentration of 0.3 to 10 % by weight, and preferably from 0.4 to 5 % by weight, relative to the total weight of the composition.

9. Dyeing composition according to any one of Claims 1 to 8, characterized in that it has a pH between 3 and 10.5, adjusted using basifying agents such as aqueous ammonia, alkali metal carbonates, alkanolamines, the compounds of formula: 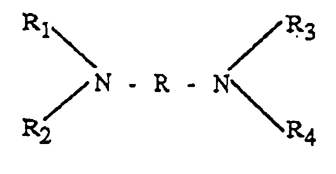 in which:
R is a propylene residue which is optionally substituted with a hydroxyl group or a C₁-C₄ alkyl radical;
R₁, R₂, R₃ and R₄, simultaneously or independently of each other, represent a hydrogen atom, a C₁-C₄ alkyl or C₁-C₄ hydroxyalkyl radical; sodium hydroxide and potassium hydroxide;
or acidifying agents such as hydrochloric, tartaric, citric and phosphoric acids.

10. Dyeing composition according to any one of Claims 1 to 9, characterized in that it contains other coupling agents chosen from α-naphthol, indole derivatives, β-keto compounds and pyrazolones, as well as their salts.

11. Dyeing composition according to any one of Claims 1 to 10, characterized in that it additionally contains direct dyes chosen from azo and anthraquinone dyes and nitro derivatives of the benzenic series.

12. Dyeing composition according to any one of Claims 1 to 11, characterized in that it additionally contains at least one adjuvant chosen from anionic, cationic, nonionic or amphoteric surface-active agents or their mixtures in proportions between 0.5 and 55 % by weight, organic solvents in proportions between 1 and 40 % by weight, thickening agents in proportions between 0.1 and 5 % by weight and anti-oxidizing agents in proportions between 0.05 and 1.5 % by weight, the proportions being calculated relative to the total weight of the composition, penetration agents, sequestering agents, perfumes, buffers, dispersing agents, conditioning agents, film-forming agents, preserving agents and opacifying agents.

13. Composition for the dyeing of keratinous fibres, and in particular human keratinous fibres such as hair, according to any one of Claims 1 to 12, which composition is ready to use, characterized in that it additionally contains an oxidizing agent and in that it has a pH between 3 and 11.

14. Ready-to-use composition according to Claim 13, characterized in that the oxidizing agent is a hydrogen peroxide solution.

15. Agent for the dyeing of keratinous fibres and in particular human keratinous fibres such as hair, characterized in that it contains at least two components: a component (A) consisting of a dyeing composition according to any one of Claims 1 to 12, and a component (B) comprising an oxidizing agent in a suitable dyeing medium.

16. Dyeing agent according to Claim 15, characterized in that the oxidizing agent is chosen from hydrogen peroxide, urea peroxide, alkali metal bromates, perborates and persulphates.

17. Process for the dyeing of keratinous fibres and in particular human keratinous fibres such as hair, characterized in that it consists in applying to the fibres a dyeing composition (A) according to any one of Claims 1 to 12, and in developing the colour in an acidic or alkaline medium using an oxidizing agent which is added just at the time of use to this composition or which is present in a composition (B) which is applied simultaneously or sequentially in a distinct manner.

18. Dyeing process according to Claim 17, characterized in that the dyeing composition according to any one of Claims 1 to 12 is mixed at the time of use with an oxidizing solution in a sufficient amount to develop a coloration, the mixture obtained is then applied to the fibres, it is left to stand for 5 to 40 minutes, preferably 15 to 30 minutes, and is then rinsed, washed with shampoo, rinsed again and dried.

19. Device containing several compartments or dyeing kit, characterized in that it contains at least two compartments, a first compartment of which contains the composition (A) as defined in any one of Claims 1 to 12, and the second compartment contains the composition (B) comprising an oxidizing agent in a suitable dyeing medium.

## Patentansprüche

1. Färbemittelzusammensetzung für Keratinfasern und insbesondere menschliche Keratinfasern, wie dem Haar, die in einem zum Färben geeigneten Medium enthält:
- mindestens ein Farbstoffvorprodukt eines Oxidationsfarbstoffes, das unter 3-Methyl-p-aminophenol, 2-Methyl-p-aminophenol und 2-Hydroxymethyl-p-aminophenol und den Additionssalzen dieser Verbindungen mit einer Säure ausgewählt ist,
- mindestens einen Kuppler, der unter den 2-Methyl-5-aminophenolen der folgenden Formel (I) und den Additionssalzen dieser Verbindungen mit einer Säure ausgewählt ist: 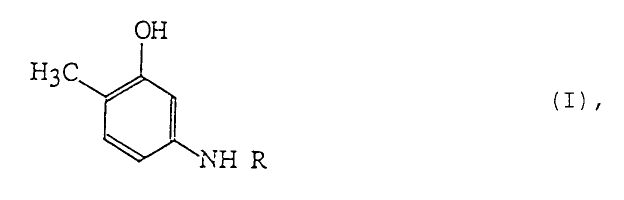 worin R Wasserstoff, Methyl, Ethyl, β-Hydroxyethyl oder γ-Hydroxypropyl bedeutet, und
- als Farbstoffvorprodukt eines Oxidationsfarbstoffes mindestens ein o-Aminophenol, das unter o-Aminophenol und 3-Acetylamino-6-aminophenol ausgewählt ist, oder die Additionssalze dieser Verbindungen mit einer Säure.

2. Färbemittelzusammensetzung nach Anspruch 1, dadurch gekennzeichnet, daß das Farbstoffvorprodukt eines Oxidationsfarbstoffes vom p-Typ 3-Methyl-p-Aminophenol oder ein Additionssalz dieser Verbindung mit einer Säure ist.

3. Färbemittelzusammensetzung nach Anspruch 1 oder 2, dadurch gekennzeichnet, daß der Kuppler 2-Methyl-5-aminophenol oder ein Additionssalz dieser Verbindung mit einer Säure ist.

4. Färbemittelzusammensetzung nach einem der Ansprüche 1 bis 3, dadurch gekennzeichnet, daß die Additionssalze mit einer Säure unter den Hydrochloriden, Sulfaten, Hydrobromiden und Tartraten ausgewählt sind.

5. Färbemittelzusammensetzung nach einem der Ansprüche 1 bis 4, dadurch gekennzeichnet, daß das 3-Methyl-p-aminophenol, das 2-Methyl-p-aminophenol und das 2-Hydroxymethyl-p-aminophenol oder deren Salze in einer Gesamtkonzentration von 0,01 bis 4 Gew.-% und vorzugsweise von 0,1 bis 2 Gew.-%, bezogen auf das Gesamtgewicht der Färbemittelzusammensetzung, vorliegen.

6. Färbemittelzusammensetzung nach einem der Ansprüche 1 bis 5, dadurch gekennzeichnet, daß die 2-Methyl-5-aminophenole der Formel (I) oder deren Salze in einer Gesamtkonzentration von 0,005 bis 5 Gew.-% und vorzugsweise von 0,01 bis 3,5 Gew.-%, bezogen auf das Gesamtgewicht der Färbemittelzusammensetzung, vorliegen.

7. Färbemittelzusammensetzung nach einem der Ansprüche 1 bis 6, dadurch gekennzeichnet, daß das o-Aminophenol oder das 3-Acetylamino-6-aminophenol in einer Gesamtkonzentration von 0,1 bis 2 Gew.-% und vorzugsweise 0,2 bis 1 Gew.-%, bezogen auf das Gesamtgewicht der Färbemittelzusammensetzung, vorliegen.

8. Färbemittelzusammensetzung nach einem der Ansprüche 1 bis 7, dadurch gekennzeichnet, daß die Farbstoffvorprodukte der Oxidationsfarbstoffe und die Kuppler in einer Gesamtkonzentration von 0,3 bis 10 Gew.-% und vorzugsweise 0,4 bis 5 Gew.-%, bezogen auf das Gesamtgewicht der Färbemittelzusammensetzung, vorliegen.

9. Färbemittelzusammensetzung nach einem der Ansprüche 1 bis 8, dadurch gekennzeichnet, daß sie einen pH-Wert im Bereich von 3 bis 10,5 aufweist, der mit Mitteln zum Alkalischmachen, wie beispielsweise Ammoniak, Alkalicarbonaten, Alkanolaminen, Verbindungen der folgenden Formel: 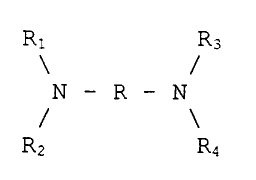 worin bedeuten:
R eine ggf. mit einer Hydroxygruppe oder einer C₁₋₄-Alkylgruppe substituierte Propylengruppe und R₁, R₂, R₃ und R₄, die identisch oder voneinander verschieden sind, ein Wasserstoffatom, eine C₁₋₄-Alkylgruppe oder eine C₁₋₄-Hydroxyalkylgruppe,
Natriumhydroxid oder Kaliumhydroxid, oder Mitteln zum Ansäuern, wie Salzsäure, Weinsäure, Citronensäure oder Phosphorsäure, auf den gewünschten Wert eingestellt wird.

10. Färbemittelzusammensetzung nach einem der Ansprüche 1 bis 9, dadurch gekennzeichnet, daß sie weitere Kuppler enthält, die unter α-Naphthol, Indolderivaten, β-Ketonverbindungen, Pyrazolonen sowie deren Salzen ausgewählt sind.

11. Färbemittelzusammensetzung nach einem der Ansprüche 1 bis 10, dadurch gekennzeichnet, daß sie ferner Direktfarbstoffe enthält, die unter Azofarbstoffen, Anthrachinonen und Nitroderivaten aus der Benzolgruppe ausgewählt sind.

12. Färbemittelzusammensetzung nach einem der Ansprüche 1 bis 11, dadurch gekennzeichnet, daß sie mindestens einen Zusatzstoff enthält, der unter den anionischen, kationischen, nichtionischen oder amphoteren grenzflächenaktiven Stoffen und deren Gemischen in Mengenanteilen von 0,5 bis 55 Gew.-%, organischen Lösungsmittel in Mengenanteilen von 1 bis 40 Gew.-%, Verdickungsmitteln in Mengenanteilen von 0,1 bis 5 Gew.-%, Antioxidantien in Mengenanteilen von 0,05 bis 1,5 Gew.-%, wobei die Mengenangaben bezogen auf das Gesamtgewicht der Zusammensetzung angegeben sind, Penetrationsmitteln, Maskierungsmitteln, Parfums, Puffern, Dispergiermitteln, Konditioniermitteln, Filmbildnern, Konservierungsmitteln und Trübungsmitteln ausgewählt ist.

13. Zusammensetzung zum Färben von Keratinfasern und insbesondere menschlichen Keratinfasern, wie dem Haar, nach einem der Ansprüche 1 bis 12, die gebrauchsfertig ist, dadurch gekennzeichnet, daß sie ferner ein Oxidationsmittel enthält und daß sie einen pH-Wert im Bereich von 3 bis 11 aufweist.

14. Gebrauchsfertige Zusammensetzung nach Anspruch 13, dadurch gekennzeichnet, daß das Oxidationsmittel eine Wasserstoffperoxidlösung ist.

15. Mittel zum Farben von Keratinfasern und insbesondere menschlichen Keratinfasern, wie dem Haar, dadurch gekennzeichnet, daß es mindestens zwei Komponenten umfaßt: eine Komponente (A), die aus einer Färbemittelzusammensetzung nach einem der Ansprüche 1 bis 12 besteht, und eine Komponente (B), die in einem zum Färben geeigneten Medium ein Oxidationsmittel enthält.

16. Mittel zum Farben nach Anspruch 15, dadurch gekennzeichnet, daß das Oxidationsmittel unter Wasserstoffperoxid, Harnstoffperoxid, Alkalimetallbromaten, Perboraten und Persulfaten ausgewählt ist.

17. Verfahren zum Färben von Keratinfasern und insbesondere menschlichen Keratinfasern, wie dem Haar, dadurch gekennzeichnet, daß es darin besteht, auf die Fasern eine Färbemittelzusammensetzung (A) nach einem der Ansprüche 1 bis 12 aufzutragen und die Farbe bei einem sauren oder alkalischen pH-Wert mit einem Oxidationsmittel zu entwickeln, das bei der Anwendung zu der Zusammensetzung gegeben wird oder das in einer Zusammensetzung (B) vorliegt, die gleichzeitig oder getrennt davon anschließend aufgetragen wird.

18. Verfahren zum Färben nach Anspruch 17, dadurch gekennzeichnet, daß die Färbemittelzusammensetzung nach einem der Ansprüche 1 bis 12 bei der Anwendung mit einer oxidierenden Lösung vermischt wird, die in einer ausreichenden Menge vorliegt, um die Färbung zu entwickeln, und anschließend das erhaltene Gemisch auf die Fasern aufgebracht und 5 bis 40 min und vorzugsweise 15 bis 30 min einwirken gelassen wird, worauf gespült, mit Haarwaschmittel gewaschen, nochmals gespült und getrocknet wird.

19. Vorrichtung mit mehreren Abteilungen oder Kit zum Färben, dadurch gekennzeichnet, daß es mindestens zwei Abteilungen aufweist, wobei eine erste Abteilung die Zusammensetzung (A) nach einem der Ansprüche 1 bis 12 und die zweite Abteilung die Zusammensetzung (B) enthält, die in einem zum Färben geeigneten Medium das Oxidationsmittel enthält.
